# EUROPÄISCHE PATENTANMELDUNG

(11) **EP 1 738 803 A1**
(43) Veröffentlichungstag der Anmeldung: **03.01.2007**
(21) Anmeldenummer: 06004604.2
(22) Anmeldetag: 07.03.2006
(51) Int. Cl.: A61Q 15/00, A61K 8/26, A61K 8/28, A61K 8/33

(54) **Inhibitoren von Staphylococcus hominis in Deodorantien und Antitranspirantien**

(30) Priorität: 16.03.2005 DE 102005012476
(71) Anmelder: Henkel Kommanditgesellschaft auf Aktien, 40589 Düsseldorf (DE)
(72) Erfinder: Banowski, Bernhard, 40597 Düsseldorf (DE); Bockmühl, Dirk, 42113 Wuppertal (DE); Scholtyssek, Regine, 40822 Mettmann (DE)

(57) **Zusammenfassung**

Gegenstand der Erfindung ist die Verwendung von mindestens einer Substanz, die den an der Körpergeruchsbildung beteiligten Keim Staphylococcus hominis inhibiert, in einer kosmetischen Deodorant- oder Antitranspirant-Zusammensetzung zur Verringerung des Körpergeruchs.

## Beschreibung

Gegenstand der Erfindung ist die Verwendung von ausgewählten Substanzen, die den Keim Staphylococcus hominis inhibieren, in einer kosmetischen Deodorant- oder Antitranspirant-Zusammensetzung zur Verringerung des Körpergeruchs.

Apokriner Schweiß stellt eine komplexe Mischung dar, die unter anderem Steroide, Cholesterin und andere Fette sowie ca. 10 % Eiweiße enthält. Körpergeruch entsteht durch den Abbau von Schweißbestandteilen durch Bakterien der Hautflora. Aus diesem Grund werden seit langem antibakterielle Substanzen in Deos verwendet. Allerdings ist bekannt, dass viele antibakterielle Wirkstoffe schlecht gegen Körpergeruch wirken. Die an der Geruchsbildung beteiligten Bakterienarten werden üblicherweise über die Kultivierung von Achselproben auf Bakteriennährböden nachgewiesen. Diese Methode hat den Nachteil, dass nicht oder nur schwer kultivierbare Arten, die an der Entstehung von Körpergeruch beteiligt sind, nicht erfasst werden. Folglich sind derartige Spezies auch nicht das Ziel von Versuchen zur Bewertung der Deodorantleistung. Außerdem lässt sich auch hinsichtlich der kultivierbaren Arten keine zuverlässige Aussage zur Menge der vorhandenen Keime treffen, da einige Arten sich besser kultivieren lassen als andere, so dass keine Aussage bezüglich ihrer Bedeutung mit Hinblick auf die Entstehung von Körpergeruch getroffen werden kann. Infolgedessen kommt es zum Einsatz vermeintlich antibakterieller Substanzen, die jedoch aufgrund des falschen Wirkspektrums nur eine unzureichende Deodorantleistung besitzen.

Die Zersetzungsprodukte des apokrinen Schweißes, die wesentlich zum Körpergeruch, insbesondere zum axillaren Körpergeruch, beitragen, lassen sich in zwei Klassen einteilen, zum einen kurzkettige, insbesondere C₄-C₁₀-Fettsäuren, die linear, verzweigt, gesättigt und ungesättigt sein können, zum anderen verschiedene Steroidhormone und deren Stoffwechselprodukte. Beispielsweise sind an dem typischen Körpergeruch, besonders an dem der Männer, die Stoffwechselprodukte der Androgene beteiligt, insbesondere Androstenol (5α-Androst-16-en-3β-ol, 5α-Androst-16-en-3α-ol) und Androstenon (5α-Androst-16-en-3-on).

Aufgabe der vorliegenden Erfindung war es, weitere Bakterien-inhibierende Wirkstoffe zu identifizieren, um eine größere Variabilität, Flexibilität und Hautverträglichkeit bei der Formulierung kosmetischer Deodorantien zu ermöglichen. Die Identifizierung bekannter kosmetischer Wirkstoffe als Inhibitoren für bestimmte geruchsbildende Bakterien ermöglicht darüber hinaus, die Dosierung dieser Wirkstoffe herunterzusetzen.

Es wurde nun gefunden, dass der Hemmung des Keims Staphylococcus hominis bei der Bekämpfung von Körpergeruch eine besondere Bedeutung zukommt.

Gegenstand der vorliegenden Erfindung ist in einer ersten Ausführungsform die Verwendung von mindestens einer Substanz, die den an der Körpergeruchsbildung beteiligten Keim Staphylococcus hominis inhibiert, in einer kosmetischen Deodorant- oder Antitranspirant-Zusammensetzung zur Verringerung des Körpergeruchs.

Unter den den an der Körpergeruchsbildung beteiligten Keim Staphylococcus hominis inhibierenden Substanzen werden erfindungsgemäß solche Substanzen verstanden, deren Wirkung zu einer statistisch signifikanten Verlangsamung oder völligen Hemmung des mikrobiellen Wachstums von Staphylococcus hominis im Vergleich zu einer unbehandelten Kontrolle führt.
Als Substanzen, die den an der Körpergeruchsbildung beteiligten Keim Staphylococcus hominis inhibieren und erfindungsgemäß in einer kosmetischen Deodorant- oder Antitranspirant-Zusammensetzung zur Verringerung des Körpergeruchs eingesetzt werden können, eignet sich eine Vielzahl von Verbindungen. Besonders bevorzugte erfindungsgemäße Verwendungen sind dadurch gekennzeichnet, dass die den an der Körpergeruchsbildung beteiligten Keim Staphylococcus hominis inhibierende(n) Substanz(en) ausgewählt ist/sind aus Derivaten von C₃-C₉-Polyolen, Phenolderivaten, monocyclischen Sesquiterpenen vom Bisabolen- oder Bisabolan-Typ, primären Alkanolen mit einer Alkylkettenlänge von C₃ - C₈ und einem Benzyl- oder Phenylsubstituenten, Aluminiumchlorohydrat, Aluminiumzirkoniumchlorohydrat, Extrakten aus Citrusfruchtsamen, den Riechstoffgemischen Protectate HR und Protectate MOD 2 und Protectate MOD 3, sowie Mischungen dieser Substanzen.

Bevorzugte erfindungsgemäße Verwendungen sind dadurch gekennzeichnet, dass die Derivate von C₃-C₉-Polyolen ausgewählt sind aus Monoalkyl-C₃-C₉-Polyolethern. Bevorzugt verwendete Monoalkyl-C₃-C₉-Polyolether sind insbesondere Monoalkylglycerinether der allgemeinen Formel R-O-CH₂-CHOH-CH₂OH, wobei R eine lineare oder verzweigte C₁-C₂₂-Alkylgruppe darstellt. R ist bevorzugt eine C₆-C₁₂-Alkylgruppe, die in 2-Position eine weitere Alkylgruppe, bevorzugt eine Methyl-, Ethyl- oder n-Propylgruppe aufweist. Besonders bevorzugt sind α-Monohexylglycerinether, α-Mono-(2-ethylhexyl)glycerinether, α-Mono-(2-ethyloctyl)glycerinether und α-Mono-(2-ethyldecyl)glycerinether. Außerordentlich bevorzugt ist α-Mono-(2-ethylhexyl)glycerinether oder kurz 2-Ethylhexylglycerinether, erhältlich unter dem Namen Sensiva SC 50 von der Firma Schülke und Mayr.

Erfindungsgemäß werden die Derivate von C₃-C₉-Polyolen in Gesamtmengen von 0,01 bis 15 Gew.-%, bevorzugt 0,05 bis 10 Gew.-% und besonders bevorzugt 0,1 bis 5 Gew.-%, jeweils bezogen auf die gesamte kosmetische Zusammensetzung, eingesetzt.
Erfindungsgemäß werden die Derivate von C₃-C₉-Polyolen, die ausgewählt sind aus Monoalkylglycerinethern der allgemeinen Formel R-O-CH₂-CHOH-CH₂OH, in Gesamtmengen von 0,01 bis 10 Gew.-%, bevorzugt 0,05 bis 5 Gew.-% und besonders bevorzugt 0,1 bis 3 Gew.-%, jeweils bezogen auf die gesamte kosmetische Zusammensetzung, eingesetzt.

Erfindungsgemäß bevorzugt verwendete Phenolderivate sind ausgewählt aus Phenoxyethanol, 4-Methoxybenzylalkohol (para-Anisalkohol), 5-Methyl-2-isopropylphenol (Thymol), Salicylsäure, Rosmarinsäure, Kaffeesäure, Tocopherolen und Tocopherylestern, insbesondere α-Tocopherol, Tocopherylacetat, Tocopherylnicotinat, Tocopherylphosphat und Tocopherylsuccinat, sowie Mischungen der vorgenannten Substanzen.
Besonders bevorzugt sind Phenoxyethanol, 4-Methoxybenzylalkohol (para-Anisalkohol) und 5-Methyl-2-isopropylphenol (Thymol) sowie Mischungen dieser Substanzen.
Erfindungsgemäß werden die Phenolderivate in Gesamtmengen von 0,001 bis 10 Gew.-%, bevorzugt 0,005 bis 5 Gew.-% und besonders bevorzugt 0,08 bis 3 Gew.-%, jeweils bezogen auf die gesamte kosmetische Zusammensetzung, eingesetzt.

Erfindungsgemäß bevorzugt verwendete monocyclische Sesquiterpene vom Bisabolen- oder Bisabolan-Typ sind ausgewählt sind aus (-)-α-Bisabolol, (R)-(E)-α-Bisabolen, (S)-(E)-α-Bisabolen, (R)-(Z)-α-Bisabolen, (S)-(Z)-α-Bisabolen, (R)-β-Bisabolen, (S)-β-Bisabolen, (E)-γ-Bisabolen, (Z)-γ-Bisabolen, Zingiberen, (R)-(-)-α-Curcumen, (S)-(+)-α-Curcumen, (S)-(+)-β-Curcumen und (S)-(+)-γ-Curcumen.
Erfindungsgemäß werden die monocyclischen Sesquiterpene vom Bisabolen- oder Bisabolan-Typ in Gesamtmengen von 0,001 bis 3 Gew.-%, bevorzugt 0,01 bis 1,0 Gew.-% und besonders bevorzugt 0,04 bis 0,5 Gew.-%, jeweils bezogen auf die gesamte kosmetische Zusammensetzung, eingesetzt.

Erfindungsgemäß bevorzugt verwendete primäre Alkanole mit einer Alkylkettenlänge von C₃ - C₈ und einem Benzyl- oder Phenylsubstituenten weisen n-Propanol-1, n-Butanol-1, n-Pentanol-1, n-Hexanol-1, n-Heptanol-1 oder n-Octanol-1 als Grundgerüst auf, das mit Methyl-, Ethyl-, n-Propyl-, iso-Propyl-, n-Butyl-, sec-Butyl- oder tert.-Butyl-Gruppen verzweigt sein kann. Weiterhin enthält das Gründgerüst in 2-Stellung, 3-Stellung, 4-Stellung, 5-Stellung, 6-Stellung, 7-Stellung oder 8-Stellung eine Benzyl- oder Phenylgruppe, die gegebenenfalls weitere Substituenten aufweisen kann, beispielsweise Methyl-, Ethyl-, n-Propyl-, iso-Propyl-, n-Butyl-, sec-Butyl- oder tert.-Butyl-Gruppen, Hydroxylgruppen oder Halogenatome, insbesondere Fluor und Chlor.
Besonders bevorzugte primäre Alkanole mit einer Alkylkettenlänge von C₃ - C₈ und einem Benzyl- oder Phenylsubstituenten sind ausgewählt aus 2-Methyl-5-phenylpentan-1-ol (mit dem Trivialnamen Rosaphen) und 2-Benzylheptan-1-ol (mit dem Trivialnamen Jasmol).
Erfindungsgemäß werden die primären Alkanole mit einer Alkylkettenlänge von C₃ - C₈ und einem Benzyl- oder Phenylsubstituenten in Gesamtmengen von 0,001 bis 10 Gew.-%, bevorzugt 0,005 bis 5 Gew.-% und besonders bevorzugt 0,03 bis 1,0 Gew.-%, jeweils bezogen auf die gesamte kosmetische Zusammensetzung, eingesetzt.

Erfindungsgemäß bevorzugt ist die Verwendung von Aluminiumchlorohydraten, insbesondere Aluminiumchlorohydrat und/oder Aluminiumzirkoniumchlorohydrat. Aluminiumchlorohydrat ist ein gebräuchlicher Antitranspirant-Wirkstoff, der als Adstringens allerdings in höheren Konzentrationen von 5 bis 25 Gew.-% eingesetzt werden muss, um in für den Verbraucher akzeptabler Weise wirksam zu sein. Für die Inhibierung des Keims Staphylococcus hominis genügen dagegen niedrigere Konzentrationen von 0,2 bis 3 Gew.-%, bevorzugt 0,5 bis 3 Gew.-% und besonders bevorzugt 1,0 bis 2,0 Gew.-%, jeweils bezogen auf die gesamte kosmetische Zusammensetzung.

Erfindungsgemäß wird Aluminiumchlorohydrat und/oder Aluminiumzirkoniumchlorohydrat in Mengen von 0,1 bis 25 Gew.-%, bevorzugt 0,5 bis 10,0 und besonders bevorzugt 1,0 bis 5,0 Gew.-%, jeweils bezogen auf die gesamte kosmetische Zusammensetzung, eingesetzt. Antitranspirantprodukte mit einem hohen Wirkstoffanteil von bis zu 25 Gew.% besitzen auch diese Eigenschaft.

Weitere bevorzugt verwendete Inhibitoren von Staphylococcus hominis sind ausgewählt aus Extrakten aus Citrusfruchtsamen. Erfindungsgemäß bevorzugte Citrusfruchtsamen stammen dabei von allen Citrusarten, besonders bevorzugt von Citrus sinensis, C. paradisi, C. aurantium, C. aurantifolia, C. reticulata, C. grandis, C. limonia und C. medica, außerordentlich bevorzugt von C. aurantium, C. sinensis, C. paradisi, C. limonia und C. reticulata. Besonders bevorzugt sind wässrige, ethanolische, propylenglycolische, Glycerin- oder butylenglycolische Extrakte. Erfindungsgemäß besonders bevorzugt verwendet werden Citrusfruchtsamen-Extrakte, die durch einen Druckaufschluss von etwa 12 - 72 Stunden, bevorzugt etwa 48 Stunden Dauer in Glycerin bei Temperaturen von etwa 50 - 150°C und anschließendem Zentrifugieren und Filtrieren erhältlich sind. Besonders bevorzugte Citrusfruchtsamen-Extrakte sind erhältlich von Centroflora (Brasilien) unter der Bezeichung Citrus Liquid Extract.
Erfindungsgemäß bevorzugt wird der Extrakt aus Citrusfruchtsamen in einer Gesamtmenge von 0,01 bis 5 Gew.-%, bevorzugt 0,05 bis 1 und besonders bevorzugt 0,1 bis 0,5 Gew.-% Aktivsubstanz, jeweils bezogen auf die gesamte erfindungsgemäß verwendete kosmetische Zusammensetzung, eingesetzt.

Das erfindungsgemäß bevorzugte Riechstoffgemisch Protectate HR der Firma Symrise enthält eine Mischung aus Phenoxyethanol, 2-Methyl-5-phenylpentan-1-ol mit dem Trivialnamen Rosaphen, 2-Benzylheptan-1-ol mit dem Trivialnamen Jasmol, 4-Methoxybenzylalkohol (Anisalkohol) und 5-Methyl-2-isopropylphenol (Thymol). Das erfindungsgemäß bevorzugte Riechstoffgemisch Protectate MOD 2 der Firma Symrise enthält eine Mischung aus Phenoxyethanol, 2-Methyl-5-phenylpentan-1-ol und 2-Benzyl-heptan-1-ol und ist frei von Anisalkohol und Thymol. Das erfindungsgemäß bevorzugte Riechstoffgemisch Protectate MOD 3 der Firma Symrise enthält eine Mischung aus Phenoxyethanol und 2-Benzylheptan-1-ol und ist frei von 2-Methyl-5-phenylpentan-1-ol, Anisalkohol und Thymol.
Erfindungsgemäß werden die Riechstoffgemische Protectate HR, Protectate Mod 2 oder Protectate MOD 3 in Gesamtmengen von 0,001 bis 5 Gew.-%, bevorzugt 0,05 bis 2 Gew.-% und besonders bevorzugt 0,1 bis 1,0 Gew.-%, jeweils bezogen auf die gesamte kosmetische Zusammensetzung, eingesetzt.

Neben der Verwendung der genannten Einzelverbindungen ist die Verwendung von Kombinationen von zwei, drei und mehr Inhibitoren des an der Körpergeruchsbildung beteiligten Keims Staphylococcus hominis erfindungsgemäß besonders bevorzugt. Besonders bevorzugt sind Mischungen aus 0,1 - 2,0 Gew.-%, besonders bevorzugt 0,25 - 1,5 Gew.-% und außerordentlich bevorzugt 0,5 - 1,0 Gew.-% α-Mono-(2-ethylhexyl)glycerinether, 0,1 - 2,5 Gew.-%, insbesondere 0,5 - 2,0 Gew.-% und außerordentlich bevorzugt 1,0 - 1,5 Gew.-% Phenoxyethanol und 0,05 - 1,0 Gew.-%, bevorzugt 0,125 - 0,7 Gew.-% und besonders bevorzugt 0,2 - 0,4 Gew.-% Protectate MOD 2 oder Protectate MOD 3, jeweils bezogen auf das Gewicht der gesamten kosmetischen Zusammensetzung. Im Falle von treibgashaltigen, als Aerosol versprühbaren Zusammensetzungen beziehen sich die vorgenannten und alle im folgenden genannten Mengenangaben jeweils auf das Gewicht der treibgashaltigen Zusammensetzung, sofern nichts anderes vermerkt ist.
Besonders bevorzugt sind Mischungen aus 0,1 - 2,0 Gew.-%, besonders bevorzugt 0,4 - 1,5 Gew.-% und außerordentlich bevorzugt 0,5 - 1,0 Gew.-% α-Mono-(2-ethylhexyl)glycerinether, 0,1 - 2,5 Gew.-%, insbesondere 0,8 - 2,0 Gew.-% und außerordentlich bevorzugt 1,0 - 1,5 Gew.-% Phenoxyethanol und 0,03 - 1,0 Gew.-%, bevorzugt 0,15 - 0,5 Gew.-% und besonders bevorzugt 0,3 - 0,4 Gew.-% Jasmol, jeweils bezogen auf das Gewicht der gesamten kosmetischen Zusammensetzung.
Weiterhin sind besonders bevorzugt Mischungen aus 0,1 - 2,0 Gew.-%, besonders bevorzugt 0,4 - 1,5 Gew.-% und außerordentlich bevorzugt 0,5 - 1,0 Gew.-% α-Mono-(2-ethylhexyl)glycerinether, 0,001 - 3,0 Gew.-%, insbesondere 0,04 - 1,0 Gew.-% und besonders bevorzugt 0,06 - 0,1 Gew.-% (-)-α-Bisabolol und 0,1 - 2,5 Gew.-%, insbesondere 0,8 - 2,0 und außerordentlich bevorzugt 1,0 - 1,5 Gew.-% Phenoxyethanol, jeweils bezogen auf das Gewicht der gesamten kosmetischen Zusammensetzung.

Weitere erfindungsgemäß besonders bevorzugt verwendete Zusammensetzungen sind durch einen Gehalt an Triethylcitrat gekennzeichnet. Damit ist die Verwendung von Mischungen aus 0,1 - 1,5 Gew.-%, bevorzugt 0,25 - 1,0 Gew.-% α-Mono-(2-ethylhexyl)-glycerinether, 0,5 - 2,0 Gew.-% Phenoxyethanol, 0,125 - 1,0 Gew.-% Protectate MOD 2 oder MOD 3 und 0,1 - 6 Gew.-%, insbesondere 1,5 - 4 Gew.-% Triethylcitrat, jeweils bezogen auf das Gewicht der gesamten kosmetischen Zusammensetzung, erfindungsgemäß besonders bevorzugt. Auch die Verwendung von Mischungen aus 0,1 - 1,2 Gew.-%, bevorzugt 0,25 - 1,0 Gew.-% α-Mono-(2-ethylhexyl)glycerinether, 0,001 - 3,0 Gew.-% (-)-α-Bisabolol, 0,5 - 2,0 Gew.-% Phenoxyethanol und 0,1 - 6 Gew.-%, insbesondere 0,5 - 4 Gew.-% und besonders bevorzugt 1,5 - 2 Gew.-% Triethylcitrat, jeweils bezogen auf das Gewicht der gesamten kosmetischen Zusammensetzung, ist erfindungsgemäß besonders bevorzugt. Auch die Verwendung von Mischungen aus 0,1 - 1,5 Gew.-%, bevorzugt 0,25 - 1,0 Gew.-% α-Mono-(2-ethylhexyl)glycerinether, 0,5 - 2,0 Gew.% Phenoxyethanol, 0,03 - 0,75 Gew.-%, bevorzugt 0,075 - 0,5 Gew.-% Jasmol und 0,1 - 6 Gew.-%, bevorzugt 0,5 - 4 Gew.-% und besonders bevorzugt 1,5 - 2 Gew.-% Triethylcitrat, jeweils bezogen auf das Gewicht der gesamten kosmetischen Zusammensetzung, ist erfindungsgemäß besonders bevorzugt.

Ein weiterer Gegenstand der vorliegenden Erfindung ist ein Verfahren zur Verringerung von Körpergeruch mittels Inhibierung des an der Körpergeruchsbildung beteiligten Keims Staphylococcus hominis auf der Haut, dadurch gekennzeichnet, dass eine kosmetische Deodorant- oder Antitranspirant-Zusammensetzung, enthaltend mindestens eine den Keim Staphylococcus hominis inhibierende Substanz, auf die Haut, insbesondere auf die Haut der Achselhöhlen, aufgetragen wird.

Bevorzugte erfindungsgemäße Verfahren sind dadurch gekennzeichnet, dass die an der Körpergeruchsbildung beteiligte(n) Staphylococcus hominis inhibierende(n) Substanz(en) ausgewählt ist/sind aus Derivaten von C₃-C₉-Polyolen, Phenolderivaten, monocyclischen Sesquiterpenen vom Bisabolen- oder Bisabolan-Typ, primären Alkanolen mit einer Alkylkettenlänge von C₃ - C₈ und einem Benzyl- oder Phenylsubstituenten, insbesondere Jasmol, Aluminiumchlorohydrat, Aluminiumzirkoniumchlorohydrat, Extrakten aus Citrusfruchtsamen, den Riechstoffgemischen Protectate HR, Protectate MOD 2 und Protectate MOD 3 der Firma Symrise sowie Mischungen dieser Substanzen.

Die kosmetischen Deodorant- oder Antitranspirant-Zusammensetzungen, die die erfindungsgemäß verwendeten Inhibitoren von Staphylococcus hominis enthalten, können bevorzugt als Puder, in Stiftform, als Aerosolspray, Pumpspray, flüssige und gelförmige Roll on-Applikation, Creme, Gel und als getränktes flexibles Substrat vorliegen.
Deodorant- oder Antitranspirant-Stifte können in gelierter Form, auf wasserfreier Wachsbasis und auf Basis von W/O-Emulsionen und O/W-Emulsionen vorliegen. Gelstifte können auf der Basis von Fettsäureseifen, Dibenzylidensorbitol, N-Acylaminosäureamiden, 12-Hydroxystearinsäure und anderen Gelbildnern hergestellt werden.
Aerosolsprays, Pumpsprays, Roll on-Applikationen und Cremes können bevorzugt als Wasser-in-Öl-Emulsion, Öl-in-Wasser-Emulsion, Siliconöl-in-Wasser-Emulsion, Wasser-in-Siliconöl-Emulsion, Wasser-in-Öl-Mikroemulsion, Öl-in-Wasser-Mikroemulsion, Siliconöl-in-Wasser-Mikroemulsion, Wasser-in-Siliconöl-Mikroemulsion, wasserfreie Suspension, alkoholische und hydroalkoholische Lösung, wässriges Gel und als Öl vorliegen. Alle genannten Zusammensetzungen können verdickt sein, beispielsweise auf der Basis von Fettsäureseifen, Dibenzylidensorbitol, N-Acylaminosäureamiden, 12-Hydroxystearinsäure, Polyacrylaten vom Carbomer- und Carbopol-Typ, Polyacrylamiden und Polysacchariden, die chemisch und/oder physikalisch modifiziert sein können.
Die Emulsionen und Mikroemulsionen können transparent, translucent oder opak sein.

Die kosmetischen Deodorant- oder Antitranspirant-Zusammensetzungen, die die erfindungsgemäß verwendeten Inhibitoren von Staphylococcus hominis enthalten, können in bevorzugten Ausführungsformen Fettstoffe enthalten. Unter Fettstoffen sind Fettsäuren, Fettalkohole, natürliche und synthetische kosmetische Ölkomponenten sowie natürliche und synthetische Wachse zu verstehen, die sowohl in fester Form als auch flüssig in wässriger oder öliger Dispersion vorliegen können.
Als Fettsäuren können eingesetzt werden lineare und/oder verzweigte, gesättigte und/oder ungesättigte C₈₋₃₀-Fettsäuren. Bevorzugt sind C₁₀₋₂₂-Fettsäuren. Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, lsostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt ist der Einsatz von Stearinsäure. Die eingesetzten Fettsäuren können eine oder mehrere Hydroxygruppen tragen. Bevorzugte Beispiele hierfür sind die a-Hydroxy-C₈-C₁₈-Carbonsäuren sowie 12-Hydroxystearinsäure.
Die Einsatzmenge beträgt dabei 0,1 - 15 Gew.-%, bevorzugt 0,5 - 10 Gew.-%, besonders bevorzugt 1 - 7 Gew.-%, jeweils bezogen auf die gesamte Zusammensetzung.

Als Fettalkohole können eingesetzt werden gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar im Sinne der Erfindung sind z.B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.

Für Stiftformulierungen werden häufig Wachse verwendet. Als natürliche oder synthetische Wachse können erfindungsgemäß eingesetzt werden feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.

Weiterhin geeignet sind die Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs^{®}) oder Polyolen mit 2 - 6 C-Atomen, Fettsäuremonoalkanolamide mit einem C₁₂₋₂₂-Acylrest und einem C₂₋₄-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/ oder ungesättigten, verzweigten und/ oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen, sofern die einzelnen Wachskomponenten oder ihre Mischung bei Raumtemperatur fest sind.
Besonders bevorzugt ist, die Wachskomponenten zu wählen aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen, sofern die Wachskomponente oder die Gesamtheit der Wachskomponenten bei Raumtemperatur fest sind. Insbesondere vorteilhaft können die Wachskomponenten aus der Gruppe der C₁₆₋₃₆-Alkylstearate, der C₁₀₋₄₀-Alkylstearate, der C₂₋₄₀-Alkylisostea-rate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse, zum Beispiel Stearyltrimethylsilan/Stearylalkohol sind gegebenenfalls vorteilhaft. Besonders bevorzugte Wachskomponenten sind die Ester aus gesättigten, einwertigen C₂₀₋C₆₀-Alkoholen und gesättigten C₈-C₃₀-Monocarbonsäuren, insbesondere ein C₂₀-C₄₀-Alkylstearat bevorzugt, das unter den Namen Kesterwachs^{®} K82H (INCl: C20-40 Alkyl Stearate) und K62 (INCl: Cetearyl behenate) von der Firma Koster Keunen Inc. erhältlich ist. Das Wachs oder die Wachskomponenten sollten bei 25° C fest sein, jedoch im Bereich von 35 - 95°C schmelzen, wobei ein Bereich von 45 - 85 °C bevorzugt ist.
Natürliche, chemisch modifizierte und synthetische Wachse können allein oder in Kombination eingesetzt werden.
Die Wachskomponenten sind in einer Menge von 0,1 bis 40 Gew.-%, bezogen auf die Gesamtzusammensetzung, vorzugsweise 0,5 bis 30 Gew.-% und insbesondere 1 - 15 Gew.-% enthalten.

Weitere erfindungsgemäß bevorzugt verwendete Zusammensetzungen sind dadurch gekennzeichnet, dass sie wenigstens ein unpolares oder polares flüssiges Öl, das natürlich oder synthetisch sein kann, enthalten.
Die polare Ölkomponente ist bevorzugt ausgewählt aus pflanzlichen Ölen, z. B. Sonnenblumenöl, Olivenöl, Sojaöl, Rapsöl, Mandelöl, Jojobaöl und den flüssigen Anteilen des Kokosöls sowie synthetischen Triglyceridölen, aus Esterölen, das heißt den Estern von C₆₋₃₀-Fettsäuren mit C₂₋₃₀-Fettalkoholen, aus Dicarbonsäureestern wie Di-n-butyladipat, Di-(2-ethylhexyl)-adipat und Di-(2-ethylhexyl)-succinat sowie Diolestern wie Ethylenglykoldioleat und Propylenglykoldi(2-ethylhexanoat), aus symmetrischen, unsymmetrischen oder cyclischen Estern der Kohlensäure mit Fettalkoholen, beispielsweise beschrieben in der DE-OS 197 56 454, Glycerincarbonat oder Dicaprylylcarbonat (Cetiol^{®} CC), aus Mono-, Di- und Trifettsäureestern von gesättigten und/oder ungesättigten linearen und/oder verzweigten Fettsäuren mit Glycerin, z. B. Cutina^{®} MD, aus verzweigten Alkanolen, z. B. Guerbet-Alkoholen mit einer einzigen Verzweigung am Kohlenstoffatom 2 wie 2-Hexyldecanol, 2-Octyldodecanol, Isotridecanol und lsohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethern oder Di-n-alkylethern mit jeweils insgesamt 12 - 24 C-Atomen, insbesondere Di-n-octylether (Cetiol^{®}OE ex Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol^{®} APM), PPG-14-Butylether (Ucon Fluid^{®} AP), PPG-15-Stearylether (Arlamol^{®} E), PPG-9-Butylether (Breox^{®} B25) und PPG-10-Butandiol (Macol^{®} 57).
Die unpolare Ölkomponente kann ausgewählt sein aus flüssigen Paraffinölen, Isoparaffinölen, z. B. Isohexadecan und Isoeicosan, aus synthetischen Kohlenwasserstoffen, z. B. 1,3-Di-(2-ethyl-hexyl)-cyclohexan (Cetiol^{®} S), sowie aus flüchtigen und nichtflüchtigen Siliconölen, die cyclisch, wie z. B. Decamethylcyclopentasiloxan und Dodecamethylcyclohexasiloxan, oder linear sein können, z. B. Polydialkylsiloxane, wie bevorzugt Hexamethyldisiloxan (L₂), Octamethyltrisiloxan (L₃), 3-Hexylheptamethyltrisiloxan (z. B. DC 2-1731 von Dow Corning), Decamethyltetrasiloxan (L₄), Dodecamethylpentasiloxan (L₅), Polyalkylarylsiloxane, z. B. Poly(methylphenylsiloxan), sowie Mischungen der vorgenannten Substanzen, insbesondere bevorzugt Mischungen aus L₃ und L₄, wie sie unter der Bezeichnung DC 2-1184 von Dow Corning erhältlich ist, weiterhin bevorzugt die unter der Bezeichnung Dow Corning^{®} 200 Fluid erhältlichen Polymere mit unterschiedlicher Kettenlänge und Viskosität (INCI-Bezeichnung: Dimethicone), insbesondere mit Viskositäten von 0,65 cSt, 1,0 cSt, 1,5 cSt, 2,0 cSt, 5,0 cSt, 6,0 cSt, 10 cSt, 100 cSt und höhere Viskositäten. Weitere bevorzugte Siliconöle sind im Handel erhältlich z. B. unter der Bezeichnung Dow Corning^{®} 190, 200, 244, 245, 344 oder 345 und Baysilon^{®} 350 M.

Weitere erfindungsgemäß bevorzugt verwendete Zusammensetzungen sind dadurch gekennzeichnet, dass sie wenigstens einen wasserlöslichen Alkohol enthalten. Unter Wasserlöslichkeit versteht man erfindungsgemäß, dass sich wenigstens 5 Gew.-% des Alkohols bei 20 °C klar lösen oder aber - im Falle langkettiger oder polymerer Alkohole - durch Erwärmen der Lösung auf 50 °C bis 60 °C in Lösung gebracht werden können. Geeignet sind je nach Darreichungsform einwertige Alkohole wie z. B. Ethanol, Propanol oder Isopropanol. Weiterhin geeignet sind wasserlösliche Polyole. Hierzu zählen wasserlösliche Diole, Triole und höherwertige Alkohole sowie Polyethylenglycole. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Hexandiole wie z. B. 1,6-Hexandiol. Weiterhin bevorzugt geeignet sind Glycerin und insbesondere Diglycerin und Triglycerin, 1,2,6-Hexantriol sowie die Polyethylenglycole (PEG) PEG-400, PEG-600, PEG-1000, PEG-1550, PEG-3000 und PEG-4000.
Die Menge des Alkohols oder des Alkohol-Gemisches in den erfindungsgemäß bevorzugt verwendeten Zusammensetzungen beträgt 1 - 50 Gew.-% und vorzugsweise 5-40 Gew.-%, bezogen auf die gesamte Zusammensetzung. Erfindungsgemäß kann sowohl ein Alkohol als auch ein Gemisch mehrerer Alkohole eingesetzt werden.

Weitere erfindungsgemäß bevorzugt verwendete Zusammensetzungen sind dadurch gekennzeichnet, dass sie im wesentlichen wasserfrei sind, das heißt maximal 5 Gew.-%, bevorzugt maximal 1 Gew.-% Wasser, bezogen auf das Gewicht der gesamten kosmetischen Zusammensetzung, enthalten.

In den bevorzugt verwendeten wasserhaltigen Darreichungsformen beträgt der Wassergehalt 5 - 98 Gew.-%, bevorzugt 10 - 90 und besonders bevorzugt 15 - 85 Gew.-%, bezogen auf die gesamte kosmetische Zusammensetzung.

Weitere erfindungsgemäß bevorzugt verwendete Zusammensetzungen sind dadurch gekennzeichnet, dass sie wenigstens ein hydrophil modifiziertes Silicon enthalten. Hydrophil modifizierte Silicone ermöglichen die Formulierung hochtransparenter Zusammensetzungen, reduzieren die Klebrigkeit und hinterlassen ein frisches Hautgefühl. Unter hydrophil modifizierten Siliconen werden erfindungsgemäß Polyorganosiloxane mit hydrophilen Substituenten verstanden, die die Wasserlöslichkeit der Silicone bedingen. Erfindungsgemäß wird unter Wasserlöslichkeit verstanden, dass sich wenigstens 2 Gew.-% des mit hydrophilen Gruppen modifizierten Silicons in Wasser bei 20 °C lösen. Entsprechende hydrophile Substituenten sind beispielsweise Hydroxy-, Polyethylenglycol- oder Polyethylenglycol/Polypropylenglycol-Seitenketten sowie ethoxylierte Ester-Seitenketten. Erfindungsgemäß bevorzugt geeignet sind hydrophil modifizierte Silicon-Copolyole, insbesondere solche Verbindungen mit der früheren INCI-Bezeichnung Dimethicone Copolyol, die beispielsweise von Wacker-Chemie unter der Bezeichnung Belsil^{®} DMC 6031, Belsil^{®} DMC 6032, Belsil^{®} DMC 6038 oder Belsil^{®} DMC 3071 VP bzw. von Dow Corning unter der Bezeichnung DC 2501 bzw. von Degussa unter der Bezeichnung Abil EM 97 im Handel sind. Besonders bevorzugt geeignet ist die Verwendung von Belsil^{®} DMC 6038 bzw. Abil EM 97, da es die Formulierung hochtransparenter Zusammensetzungen ermöglicht, die beim Verbraucher eine höhere Akzeptanz erreichen. Erfindungsgemäß kann auch ein beliebiges Gemisch dieser Silicone eingesetzt werden.
Weitere hydrophil modifizierte Silicon-Copolyole sind ausgewählt aus den Poly-(C₂-C₃)-alkylenglycol-modifizierten Siliconen, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11-12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3-30 und besonders bevorzugt 12-20, insbesondere 14 - 18, stehen), Bis-PEG/PPG-c/d Dimethicone (wobei c und d unabhängig voneinander für Zahlen von 10-25, bevorzugt 14-20 und besonders bevorzugt 14 - 16, stehen) und Bis-PEG/PPG-e/f PEG/PPG g/h Dimethicone (wobei e, f, g und h unabhängig voneinander für Zahlen von 10-20. bevorzugt 14-18 und besonders bevorzugt 16, stehen). Bevorzugt sind PEG/PPG-18/18 Dimethicone, das in einer 1:9-Mischung mit Cyclomethicone unter der Bezeichnung DC 3225 C bzw. DC 5225 C von Dow Corning im Handel erhältlich ist, sowie Bis-PEG/PPG-14/14 Dimethicone, das in einer Mischung mit Cyclomethicone unter der Bezeichnung Abil EM 97 (Goldschmidt) im Handel erhältlich ist.
Weiterhin bevorzugt sind Poly-(C₂-C₃)alkylenglycol-modifizierte Silicone, die mit C₄-C₁₈₋Alkylgruppen hydrophob modifiziert sind, besonders bevorzugt Cetyl PEG/PPG-10/1 Dimethicone (früher: Cetyl Dimethicone Copolyol, erhältlich unter der Bezeichnung Abil EM 90), weiterhin Alkyl Methicone Copolyole und Alkyl Dimethicone Ethoxy Glucoside.
Die Menge des hydrophil modifizierten Silicons oder des Alkohol-Gemisches in den bevorzugt verwendeten erfindungsgemäßen Zusammensetzungen beträgt 0,1 - 10 Gew.-%, bevorzugt 0,5 - 8 Gew.-% und besonders bevorzugt 1 - 6 Gew.-%, bezogen auf das Gesamtgewicht der Zusammensetzung.

Weitere erfindungsgemäß bevorzugt verwendete Zusammensetzungen sind dadurch gekennzeichnet, dass sie wenigstens ein wasserlösliches Tensid enthalten. Als wasserlösliche Tenside eignen sich grundsätzlich alle in Wasser bei 20°C zu mindestens 1 Gew.-% löslichen Tenside. Obwohl die Struktur und lonogenität an sich unerheblich sind, scheinen nichtionische Tenside, insbesondere die bei Normaltemperatur (20°C) festen Anlagerungsprodukte des Ethylenoxids an Fettstoffmoleküle mit wenigstens einer alkoxylierbaren Gruppe, bevorzugt geeignet zu sein. Solche geeigneten Tenside sind z.B. die Anlagerungsprodukte von 5 - 50 Mol Ethylenoxid an lineare Fettalkohole mit 16 - 22 C-Atomen, an Fettsäuren mit 5 - 22 C-Atomen, an Fettsäurealkanolamide, an Fettsäuremonoglyceride, an Sorbitan-Fettsäuremonoester, an Fettsäurealkanolamide, an Fettsäureglyceride, z.B. an gehärtetes Rizinusöl, an Methylglucosidmonofettsäureester und Gemische davon.

Weitere erfindungsgemäß bevorzugt verwendete Zusammensetzungen sind dadurch gekennzeichnet, dass sie in einer bevorzugten Ausführungsform mindestens einen Antitranspirant-Wirkstoff enthalten. Als Antitranspirant-Wirkstoffe eignen sich wasserlösliche adstringierende metallische Salze, insbesondere anorganische und organische Salze des Aluminiums, Zirkoniums und Zinks bzw. beliebige Mischungen dieser Salze. Erfindungsgemäß wird unter Wasserlöslichkeit eine Löslichkeit von wenigstens 5 g Aktivsubstanz pro 100 g Lösung bei 20 °C verstanden. Erfindungsgemäß verwendbar sind beispielsweise Alaun (KAI(SO₄)₂ · 12 H₂O) Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat und Aluminium-Zirkonium-Chlorohydrat-Glycin-Komplexe. Bevorzugt enthalten die Zusammensetzungen ein adstringierendes Aluminiumsalz, insbesondere Aluminiumchlorohydrat, und/oder eine Aluminium-Zirkonium-Verbindung. Die Antitranspirant-Wirkstoffe werden bei wässrigen Applikationen als wässrige Lösungen eingesetzt. In wasserfreien Zusammensetzungen werden die Antitranspirant-Wirkstoffe in fester Form eingesetzt. Sie sind in den erfindungsgemäßen Zusammensetzungen in einer Menge von 1 - 40 Gew.-%, vorzugsweise 5 - 30 Gew.-% und insbesondere 10 - 25 Gew.-% enthalten (bezogen auf die Menge der Aktivsubstanz in der Gesamtzusammensetzung bzw. bei aerosolförmigen Produkten bezogen auf das treibmittelfreie Konzentrat der Zusammensetzung). Aluminiumchlorohydrate werden beispielsweise pulverförmig als Micro Dry^{®} Ultrafine von Reheis, als Chlorhydrol^{®}, in aktivierter Form als Reach^{®} 501 von Reheis sowie in Form einer wäßrigen Lösung als Locron^{®} L von Clariant vertrieben. Unter der Bezeichnung Reach^{®} 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal^{®} 36G im Handel sind, ist erfindungsgemäß besonders vorteilhaft. Einsetzbar sind auch die oben genannten Antitranspirant-Wirkstoffe, die durch einen modifizierten Herstellprozess eine erhöhte schweißreduzierende Wirkung besitzen, im Handel erhältlich z. B. unter der Bezeichnung REACH 101 oder REACH 103.

Weitere erfindungsgemäß bevorzugt verwendete Zusammensetzungen sind dadurch gekennzeichnet, dass sie mindestens einen keimhemmenden oder desodorierenden Wirkstoff enthalten, dessen Wirkung auf einem anderen Mechanismus als der Hemmung von Staphylococcus hominis beruht. Bevorzugt sind insbesondere Organohalogenverbindungen sowie -halogenide, quartäre Ammoniumverbindungen, eine Reihe von Pflanzenextrakten und Zinkverbindungen. Besonders bevorzugt sind Chlorhexidin und Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar.
Weitere antibakteriell wirksame Deodorant-Wirkstoffe sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester.

Weitere erfindungsgemäß bevorzugt verwendete Zusammensetzungen sind dadurch gekennzeichnet, dass sie mindestens einen weiteren Enzyminhibitor gegen Körpergeruch, z. B. Inhibitoren für Lipase, Arylsulfatase, β-Glucuronidase, Aminoacylase etc. enthalten. Auch Kombinationen mit Antioxidantien sind bevorzugt.

Weitere erfindungsgemäß bevorzugt verwendete Zusammensetzungen sind dadurch gekennzeichnet, dass sie mindestens eine den Haarwuchs inhibierende Substanz enthalten. Geeignete Substanzen, die den Haarwuchs inhibieren, sind insbesondere ausgewählt aus Eflornithin, Wirkstoffkombinationen aus Sojaproteinhydrolysat, Harnstoff, Menthol, Salicylsäure und Extrakten aus Hypericum Perforatum, Hamamelis Virginiana, Arnica Montana und der Rinde von Salix Alba, wie sie beispielsweise und bevorzugt in dem Rohstoff Pilinhib^{®} Veg LS 9109 von Laboratoires Serobiologiques mit der INCI-Deklaration "Propylene glycol, Hydrolyzed Soy Protein, Hypericum Perforatum Extract, Hamamelis Virginiana Extract, Arnica Montana Flower Extract, Urea, Salix Alba Bark Extract, Menthol, Salicylic acid" enthalten ist, weiterhin Wirkstoffkombinationen aus Extrakten aus Epilobium Angustifolium, den Samen von Cucurbita pepo (Kürbis) und den Früchten von Serenoa serrulata, wie sie beispielsweise und bevorzugt in dem Rohstoff ARP 100 von Greentech S.A./Rahn mit der INCI-Deklaration "Water, Alcohol, Serenoa Serrulata Fruit Extract, Epilobium Angustifolium Extract, Cucurbita Pepo (Pumpkin) Seed Extract" enthalten sind, weiterhin Wirkstoffkombinationen aus Xylitol und Extrakten von Citrus Medica Limonum (Lemon) Fruit, Carica Papaya (Papaya) und Olivenblättern, wie sie beispielsweise und bevorzugt in dem Rohstoff Xyleine von Impag / Seporga mit der INCI-Deklaration "Xylitol and Citrus Medica Limonum (Lemon) Fruit Extract and Carica Papaya (Papaya) Fruit Extract and Olea europaea (olive) leaf extract" enthalten sind, weiterhin Wirkstoffkombinationen aus Humulus Lupulus, Viscum Album, Salvia Officinalis, Carica Papaya und Thuya Occidentalis, wie sie beispielsweise und bevorzugt in dem Rohstoff Plantafluid Komplex AH der Firma Plantapharm mit der INCI-Deklaration "Aqua, Propylene Glycol, Humulus Lupulus, Viscum Album, Salvia Officinalis, Carica Papaya, Thuya Occidentalis" enthalten sind, sowie Extrakten aus Larrea divaricata, wie sie beispielsweise und bevorzugt in dem Rohstoff Capislow von Sederma, der Lecithinvesikel mit einem hydroglycolischen Extrakt aus Larrea divaricata enthält, enthalten sind.
Erfindungsgemäß bevorzugt verwendete Zusammensetzungen enthalten mindestens eine den Haarwuchs inhibierende Substanz vorzugsweise in einer Menge von 0,1 - 10 Gew.-%, bevorzugt 0,5 - 5 Gew.-% und besonders bevorzugt 1 - 4 Gew.-%, jeweils bezogen auf das Gewicht des Rohstoffs tel quel und das Gesamtgewicht der erfindungsgemäß verwendeten Zusammensetzung.

Erfindungsgemäß bevorzugt verwendete flüssige und gelförmige Darreichungsformen können Verdickungsmittel enthalten, z. B. Celluloseether, wie Hydroxypropylcellulose, Hydroxyethylcellulose, hydrophob modifizierte Hydroxyethylcellulose und Methylhydroxypropylcellulose, verdickende Polymere auf Basis von Polyacrylaten, die gewünschtenfalls vernetzt sein können, z. B. die Carbopoltypen oder Pemulen^{®}-Produkte, oder auf Basis von Polyacrylamiden oder sulfonsäuregruppenhaltigen Polyacrylaten, z. B Sepigel^{®} 305 oder Simulgel^{®} EG, weiterhin anorganische Verdicker, z. B. Bentonite und Hectorite (Laponite^{®}).
Weitere erfindungsgemäß bevorzugt verwendete Zusammensetzungen sind dadurch gekennzeichnet, dass sie weitere kosmetisch und dermatologisch wirksame Stoffe enthalten, wie beispielsweise entzündungshemmende Substanzen, Feststoffe, ausgewählt aus Kieselsäuren, z. B. Aerosil^{®}-Typen, Kieselgelen, Siliciumdioxid, Tonen, z. B. Bentonite oder Kaolin, Magnesiumaluminiumsilikaten, z. B. Talkum, Bornitrid, Titandioxid, das gewünschtenfalls beschichtet sein kann, gegebenenfalls modifizierten Stärken und Stärkederivaten, Cellulosepulvern und Polymerpulvern, weiterhin Pflanzenextrakte, Proteinhydrolysate, Vitamine, Parfümöle, Sebostatika, Anti-Akne-Wirkstoffe sowie Keratolytika.

Die kosmetischen Deodorant- oder Antitranspirant-Zusammensetzungen, die mindestens einen erfindungsgemäß verwendeten Inhibitor von Staphylococcus hominis enthalten, können, soweit sie flüssig vorliegen, in einer weiteren bevorzugten Ausführungsform der Erfindung auf flexible und saugfähige Träger aufgebracht und als Deodorant- oder Antitranspirant-Tücher oder Schwämmchen angeboten werden. Als flexible und saugfähige Träger im Sinne der Erfindung eignen sich z. B. Träger aus Textilfasern, Kollagen oder polymeren Schaumstoffen. Als Textilfasern können sowohl Naturfasern wie Cellulose (Baumwolle, Leinen), Seide, Wolle, Regeneratcellulose (Viskose, Rayon), Cellulosederivate als auch synthetische Fasern wie z.B. Polyester, Polyacrylnitril, Polyamid- oder Polyolefinfasern oder Mischungen solcher Fasern gewebt oder ungewebt verwendet werden. Diese Fasern können zu saugfähigen Wattepads, Vliesstoffen oder zu Geweben oder Gewirken verarbeitet sein. Auch flexible und saugfähige polymere Schaumstoffe, z. B. Polyurethanschäume und Polyamidschäume sind geeignete Substrate. Das Substrat kann eine, zwei, drei sowie mehr als drei Lagen aufweisen, wobei die einzelnen Lagen aus gleichen oder unterschiedlichen Materialien bestehen können. Jede Substratschicht kann eine homogene oder eine inhomogene Struktur mit beispielsweise verschiedenen Zonen unterschiedlicher Dichte aufweisen.

Als saugfähig im Sinne der Erfindung sind solche Trägersubstrate anzusehen, die bei 20° C wenigstens 10 Gew.-%, bezogen auf das Trockengewicht, an Wasser adsorptiv bzw. kapillar binden können. Bevorzugt eignen sich aber solche Träger, die wenigstens 100 Gew.-% Wasser adsorptiv und kapillar binden können.

Die Ausrüstung der Trägersubstrate erfolgt in der Weise, dass man die saugfähigen, flexiblen Trägersubstrate, bevorzugt aus Textilfasern, Kollagen oder polymeren Schaumstoffen mit den erfindungsgemäßen Zusammensetzungen behandelt bzw. ausrüstet und gegebenenfalls trocknet. Dabei kann die Behandlung (Ausrüstung) der Trägersubstrate nach beliebigen Verfahren, z. B. durch Aufsprühen, Tauchen und Abquetschen, Durchtränken oder einfach durch Einspritzen der erfindungsgemäßen Zusammensetzung in die Trägersubstrate erfolgen.

Erfindungsgemäß besonders bevorzugt ist weiterhin die Darreichungsform als Aerosol, wobei die kosmetische Zusammensetzung ein Treibmittel, bevorzugt ausgewählt aus Propan, Butan, Isobutan, Pentan, Isopentan, Dimethylether, Fluorkohlenwasserstoffen und Fluorchlorkohlenwasserstoffen sowie Mischungen hiervon, enthält. Auch Aerosole mit komprimierten Gasen, wie Stickstoff, Luft, Distickstoffoxid oder Kohlendioxid, sind formulierbar.

In besonders bevorzugten erfindungsgemäßen Verfahren zur Verringerung von Körpergeruch wird/werden die den an der Körpergeruchsbildung beteiligten Keim Staphylococcus hominis inhibierende(n) Substanz(en) hinsichtlich ihrer Menge und/oder ihrer Art geschlechtsspezifisch eingesetzt. Hierbei sind insbesondere bevorzugte Verfahren dadurch gekennzeichnet, dass die den an der Körpergeruchsbildung beteiligten Keim Staphylococcus hominis inhibierende(n) Substanz(en) zur Verringerung von Körpergeruch beim Mann eingesetzt wird/werden.

Die nachfolgenden Beispiele sollen den Gegenstand der Erfindung verdeutlichen, ohne ihn hierauf zu beschränken.

Durchführung der Inhibitionsversuche

Eine Luria-broth-Vorkultur (1 Gew.-% Trypton, 0,5 Gew.-% Hefeextrakt, 0,5 Gew.-% NaCl) von Staphylococcus hominis wurde mit Medium auf eine OD₆₀₀ von 0,1 (entspricht 10⁵-10⁷ cfu/ml) eingestellt und in 50 ml-Aliquots auf Erlenmeyer-Kolben verteilt. Dazu wurden die zu testenden Wirkstoffe gegeben, so dass sie in den in Tabelle 1 angegebenen Konzentrationen vorlagen, und die Kultur mit 100 Upm bei 37°C inkubiert. Öllösliche Stoffe wie Bisabolol wurden mit 0,1% Tween emulgiert und gegen eine entsprechende Kontrolle getestet. Nach 4, 8 und 24 h erfolgte eine Keimzahlbestimmung über Ausplattieren, wobei die Hemmleistung der einzelnen Wirkstoffe als RF-Wert (Differenz zur Kontrolle in log-Stufen) angegeben ist.

**Tabelle 1**

| Nachweis der Inhibierung von Staphylococcus hominis durch erfindungsgemäß verwendete Inhibitoren [Hemmleistung als Differenz zur Kontrolle in log-Stufen angegeben] | | | | | | | |
|---|---|---|---|---|---|---|---|
| Zeit [h] | (-)-α-Bisabolol (0,05 Gew.-% Aktivsubstanz) | (-)-α-Bisabolol (0,1 Gew.-% Aktivsubstanz) | Protectate HR (0,01 Gew.-% Aktivsubstanz) | Protectate HR (0,5 Gew.-% Aktivsubstanz) | Aluminiumchlorhydrat (0,5 Gew.-% Aktivsubstanz) | Sensiva SC50 (0,2 Gew.-% Aktivsubstanz) | Sensiva SC 50 (0,5 Gew.-% Aktivsubstanz) |
| 0 | 0 | 0 | 0 | 0 | 0 | 0 | 0 |
| 4 | 1,30 | 1,98 | 0,97 | 1,47 | 6,07 | 5,77 | 6,14 |
| 8 | 1,75 | 2,60 | 1,34 | 2,34 | 6,34 | 6,19 | 6,63 |

Innerhalb des Testzeitraums von 8 Stunden nahm die Keimzahl in der mit einem erfindungsgemäßen Inhibitor behandelten Kultur gegenüber der unbehandelten Kontrollkultur um 1,75 bis 6,63 log-Stufen ab.

Weiterhin wurden die Wirkstoffe mit Hilfe des Filterpapier/Oberflächenverfahrens vermessen. Der Testkeim Staphylococcus hominis wurde 24 Stunden bei 37 °C vorinkubiert. 0,3 ml Wirkstofflösung bzw. 0,3 g wirkstoffhaltiges Produkt wurden auf eine Filterpapierscheibe mit einem Durchmesser von 4,5 cm aufgebracht, anschließend wurde die Filterpapierscheibe auf den bakterienhaltigen Agar gelegt. Nach der Inkubationszeit wurde die Größe des gebildeten Hemmhofes vermessen. Bei den Lösungen, für die kein Hemmhof detektiert werden konnte, weil der Wirkstoff nicht in den Agar penetriert war, wurde zusätzlich das Wachstum unter den Filterpapierscheiben beurteilt.

Die folgenden Formulierungsbeispiele sollen den Gegenstand der Erfindung verdeutlichen, ohne ihn hierauf zu beschränken.
2. Deodorant-Aerosolsprays

| | 1 | 2 | 3 | 4 | 5 | 6 | 7 |
|---|---|---|---|---|---|---|---|
| Triethylcitrate | 1,00 | 5,00 | 6,00 | 1,50 | 3,00 | 1,00 | 5,00 |
| 2-Ethylhexylglycerin | 0,50 | 0,10 | 0,50 | 0,20 | 0,30 | 0,50 | 0,10 |
| Phenoxyethanol | 0,30 | 0,50 | 0,10 | 0,40 | 0,60 | 0,30 | 0,50 |
| Parfum | 0,50 | 1,50 | 1,00 | 1,00 | 1,00 | 0,50 | 1,50 |
| Plant Extract | 0,05 | - | 0,20 | - | 0,00 | 0,05 | - |
| Aroma | 0,50 | 0,01 | 0,10 | 0,10 | 0,05 | 0,50 | 0,01 |
| Vitamin E-Acetate | - | - | - | 0,05 | 0,10 | - | - |
| Bisabolol | - | - | 0,10 | 0,10 | - | - | - |
| Protectate MOD 2 | 0,50 | - | 0,20 | 0,10 | 0,30 | - | - |
| Protectate HR | - | 0,5 | - | - | - | - | - |
| Protectate MOD 3 | - | - | - | - | - | 0,2 | 0,5 |
| Hydrocarbon Propellant z. B. n-Butan | 85,00 | 60,00 | 70,00 | 75,00 | 70,00 | 85,00 | 60,00 |
| Alcohol Denat. | ad 100 | ad 100 | ad 100 | ad 100 | ad 100 | ad 100 | ad 100 |

3. Deodorant-Aerosolsprays

| | 1 | 2 | 3 | 4 | 5 |
|---|---|---|---|---|---|
| Isopropylmyristate | 1,00 | 10,00 | 5,00 | 2,00 | 2,00 |
| Phenoxyethanol | 0,30 | 0,50 | 0,10 | 0,20 | 0,20 |
| Parfum | 0,50 | 1,50 | 1,00 | 0,50 | 0,50 |
| Plant Extract | 0,05 | - | 0,20 | 0,50 | 0,50 |
| 2-Ethylhexylglycerin | 0,40 | 0,10 | 1,50 | 0,80 | 0,80 |
| Protectate MOD 2 | 0,20 | 0,30 | - | - | - |
| Protectate HR | - | - | 0,20 | 0,20 | - |
| Protectate MOD 3 | - | - | - | - | 0,10 |
| Hydrocarbon Propellant, z. B. n-Butan | 75,00 | 85,00 | 78,00 | 60,00 | 60,00 |
| Alcohol Denat. | ad 100 | ad 100 | ad 100 | ad 100 | ad 100 |

4. Antitranspirant-Aerosolsprays

| | 1 | 2 | 3 | 4 | 5 | 6 |
|---|---|---|---|---|---|---|
| Aluminum chlorohydrate | 4,00 | 10,00 | | | | |
| Aluminum chlorohydrate activated | | | 2,00 | 10,00 | 2,00 | 10,00 |
| Disteardimonium Hectorite / Propylene Carbonate | 0,50 | 1,50 | 0,80 | 1,20 | 0,80 | 1,20 |
| Parfum | 0,80 | 0,50 | 1,00 | 1,50 | 1,00 | 1,50 |
| Plant Extract | 0,05 | 0,50 | 0,10 | 0,20 | 0,10 | 0,20 |
| Encapsulated Parfum / Active (Fircaps*) | 1,50 | 0,10 | 1,50 | 0,10 | 1,50 | 0,10 |
| Protectate MOD 2 | 0,05 | 0,15 | - | - | - | - |
| Protectate HR | - | - | - | 0,10 | - | - |
| Protectate MOD 3 | - | - | - | - | 0,20 | 0,10 |
| Zinkgluconate | - | - | 0,05 | - | 0,05 | - |
| Citrus Liquid Extract | - | - | 0,10 | - | - | - |
| Hydrocarbon Propellant, z. B. n-Butan | 85,00 | 75,00 | 80,00 | 60,00 | 80,00 | 60,00 |
| Cyclopentasiloxane/ Cyclohexasiloxane, z.B. Dow Corning 345 | ad 100 | ad 100 | ad 100 | ad 100 | ad 100 | ad 100 |

| | | | | | | |
|---|---|---|---|---|---|---|
| * Fircaps = in Cellulose-Derivaten verkapselte Parfüm-Menthyllactat-Mischung oder allgemein in Cellulose-Derivaten verkapselte Parfüm-Cooling Agent-Mischung, erhältlich von Firmenich | | | | | | |

5. Antitranspirant-Aerosolsprays

| | 1 | 2 | 3 | 4 | 5 | 6 |
|---|---|---|---|---|---|---|
| Aluminum chlorohydrate | 4,00 | 10,00 | - | - | - | - |
| Aluminum chlorohydrate activated | - | - | 2,00 | 10,00 | 2,00 | 10,00 |
| Disteardimonium Hectorite / Propylene Carbonate | 0,50 | 1,50 | 0,80 | 1,20 | 0,80 | 1,20 |
| Parfum | 0,80 | 0,50 | 1,00 | 1,50 | 1,00 | 1,50 |
| Plant Extract | 0,05 | 0,50 | 0,10 | 0,20 | 0,10 | 0,20 |
| Aroma | 0,50 | 0,01 | 0,05 | 0,10 | 0,05 | 0,10 |
| Di-C12-13 Alkyl Malate | - | 0,50 | 0,50 | 10,00 | 0,50 | 10,00 |
| Ethylhexylpalmitat | ad 100 | 5,00 | - | - | - | - |
| Protectate MOD 2 | 0,05 | 0,15 | - | - | - | - |
| Protectate HR | - | - | 0,20 | 0,10 | - | - |
| Protectate MOD 3 | - | - | - | - | 0,20 | 0,10 |
| Zinkgluconat | - | - | 0,05 | - | 0,05 | - |
| Hydrocarbon Propellant, z. B. n-Butan | 85,00 | 75,00 | 80,00 | 60,00 | 80,00 | 60,00 |
| Cyclopentasiloxane/ Cylclohexasiloxane, z.B. Dow Corning 345 | - | ad 100 | ad 100 | ad 100 | ad 100 | ad 100 |

6. alkoholische Roll-on-Formulierungen

| | 1 | 2 | 3 | 4 | 5 | 6 | 7 |
|---|---|---|---|---|---|---|---|
| Alcohol Denat. | 35,00 | 30,00 | 28,00 | 30,00 | 40,00 | 30,00 | 40,00 |
| Aluminum Chlorhydrate 50% solution | 16,00 | 40,00 | 16,00 | 16,00 | - | 16,00 | - |
| Aluminum Zirconium Pentachlorohydrate 40% solution | - | - | - | - | 45,00 | - | 45,00 |
| Ceteareth-12 | 2,50 | 1,50 | 2,00 | 2,00 | 2,50 | 2,00 | 2,50 |
| Ceteareth-30 | 2,50 | 2,00 | 1,50 | 2,00 | 2,50 | 2,00 | 2,50 |
| Parfum | 0,70 | 1,00 | 1,50 | 1,20 | 1,20 | 1,20 | 1,20 |
| Tocopheryl Acetate | 0,05 | 0,10 | | 0,25 | 0,05 | 0,25 | 0,05 |
| Hydroxyethylcellulose | 0,50 | 0,30 | 0,40 | 0,60 | 0,50 | 0,60 | 0,50 |
| Zinc Gluconate | - | 0,10 | - | - | 0,10 | - | 0,10 |
| Plant Extract | - | - | 0,20 | 0,50 | 0,20 | 0,50 | 0,20 |
| Colours approved for Cosmetics | 0,0005 | 0,0010 | 0,0005 | 0,0100 | 0,0001 | 0,0100 | 0,0001 |
| Phenoxyethanol | - | - | - | 1,5 | - | 1,5 | - |
| 2-Ethylhexylglycerin | - | - | 0,40 | 1,0 | - | 1,0 | - |
| Protectate MOD 2 | 0,30 | 0,50 | - | - | - | - | - |
| Protectate HR | - | - | 0,20 | 0,60 | - | - | - |
| Protectate MOD 3 | - | - | - | - | 0,20 | 0,20 | 0,10 |
| Aqua | ad 100 | ad 100 | ad 100 | ad 100 | ad 100 | ad 100 | ad 100 |

7. Roll-on-Emulsionen

| | 1 | 2 | 3 | 4 | 5 | 6 |
|---|---|---|---|---|---|---|
| Steareth-2 | 2,50 | 3,00 | 3,00 | 2,80 | 2,80 | 2,80 |
| PPG-15 Stearyl Ether | 2,00 | 3,00 | 2,00 | 2,20 | 2,00 | 2,00 |
| Steareth-21 | 1,00 | 1,00 | 3,00 | 1,00 | 1,30 | 1,30 |
| Aluminium Chlorohydrate 50% solution | 40,00 | 40,00 | 40,00 | 40,00 | 40,00 | - |
| Aluminium Zirconium Tetrachlorohydrex Gly, 35% solution | - | - | - | - | - | 63,00 |
| Allantoin | 0,10 | - | 0,10 | - | 0,10 | - |
| Plant Extract | - | 0,50 | 0,20 | - | - | 0,20 |
| Tocopheryl acetate | 0,05 | 0,05 | 0,05 | 0,25 | 0,25 | 0,25 |
| Parfum | 1,00 | 1,50 | 1,30 | 0,80 | 1,00 | 1,20 |
| Phenoxyethanol | - | - | - | - | 2,0 | 2,0 |
| 2-Ethylhexylglycerin | 0,30 | - | - | - | 1,0 | 1,0 |
| Protectate MOD 2 | 0,30 | 0,50 | - | - | 0,6 | - |
| Protectate HR | - | - | 0,20 | 0,60 | - | - |
| Jasmol | - | - | - | - | - | 0,3 |
| Aqua | ad 100 | ad 100 | ad 100 | ad 100 | ad 100 | ad 100 |

8. alkoholische Deodorant-Stifte

| | 1 | 2 | 3 | 4 | 5 |
|---|---|---|---|---|---|
| Alcohol Denat. | 40,00 | 40,00 | 35,00 | 30,00 | 30,00 |
| Propylene Glycol 1,2 | 30,32 | 32,32 | 32,32 | 38,00 | 38,00 |
| Butylene Glycol 1,3 | 12,00 | 10,00 | 15,00 | 12,00 | 12,00 |
| Sodium Palmitate | 3,10 | 3,50 | 2,80 | 3,10 | 3,10 |
| Sodium Stearate | 3,10 | 3,50 | 2,80 | 3,10 | 3,10 |
| Glycerin 86% | 2,00 | 1,00 | 1,70 | - | - |
| PPG-5-Laureth-5 | 0,50 | 1,00 | 1,00 | 0,50 | 0,50 |
| (Sodium Hydroxide 50%) | (2) | (2) | (2) | (2) | (2) |
| Parfum | 1,00 | 0,60 | 1,30 | 1,00 | 1,00 |
| Octyldodecanol | 1,00 | 0,50 | 1,00 | 0,70 | 0,70 |
| Phenoxyethanol | 1,00 | 0,50 | 1,00 | 0,50 | 0,50 |
| 2-Ethylhexylglycerin | 0,50 | - | 0,30 | 1,00 | 1,00 |
| Tocopheryl acetate | 0,05 | - | 0,10 | 0,25 | 0,25 |
| Plant Extract | 0,20 | - | 0,20 | 0,50 | - |
| PEG-40 Hydrogenated Castor Oil | 0,02 | - | - | 0,10 | 0,10 |
| Protectate MOD 2 | 0,30 | 0,50 | - | - | - |
| Protectate HR | - | - | 0,20 | 0,60 | - |
| Protectate MOD 3 | - | - | - | - | 0,20 |
| Aqua | ad 100 | ad 100 | ad 100 | ad 100 | ad 100 |

9. nicht-alkoholische Deodorant-Stifte

| | 1 | 2 | 3 | 4 | 5 |
|---|---|---|---|---|---|
| PEG-8 | 40,00 | 45,00 | 50,00 | 46,00 | 46,00 |
| Sodium Palmitate | 2,50 | 2,50 | 2,50 | 2,50 | 2,50 |
| Sodium Stearate | 2,50 | 2,50 | 2,50 | 2,50 | 2,50 |
| Butylene Glycol 1,3 | 5,00 | 2,00 | 3,00 | 4,00 | 4,00 |
| PEG-14 Dimethicone | 1,00 | 2,00 | 1,50 | 1,50 | 1,50 |
| (Sodium Hydroxide 50%) | (1,5) | (1,2) | (1,4) | (1,3) | (1,3) |
| Phenoxyethanol | 1,00 | 2,00 | 0,50 | 1,00 | 1,00 |
| Parfum | 1,00 | 1,20 | 0,80 | 1,00 | 1,00 |
| 2-Ethylhexylglycerin | 0,30 | - | 0,30 | 1,00 | 1,00 |
| Steareth-10 | 0,20 | - | 0,20 | 0,20 | 0,20 |
| Plant Extract | 0,20 | 0,50 | - | 0,30 | 0,30 |
| Protectate MOD 2 | 0,30 | 0,50 | - | - | - |
| Jasmol | - | - | - | - | 0,45 |
| Protectate HR | - | - | 0,20 | 0,60 | - |
| Aqua | ad 100 | ad 100 | ad 100 | ad 100 | ad 100 |

10. Antitranspirant-Stifte

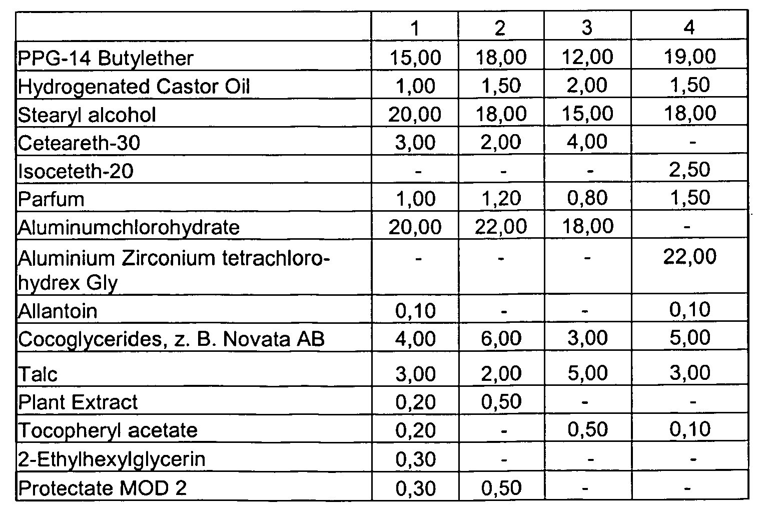

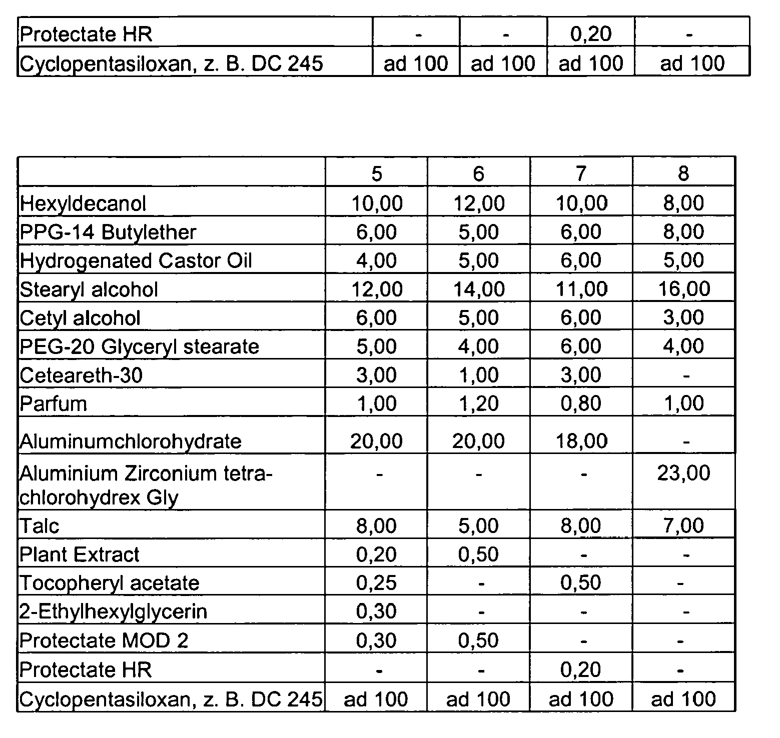

11. Deodorant-Sprays im Pumpdispenser (treibgasfrei)

| | 1 | 2 | 3 | 4 | 5 | 6 |
|---|---|---|---|---|---|---|
| Alcohol Denat. | 50,00 | 55,00 | 60,00 | 40,00 | 40,00 | 40,00 |
| Triethylcitrate | 2,50 | 3,50 | 4,00 | 3,00 | 3,00 | 3,00 |
| PEG-40 Hydrogenated Castor Oil | 1,00 | 0,50 | 0,50 | 2,00 | 2,00 | 2,00 |
| 2-Ethylhexylglycerin | 0,10 | 0,50 | - | 1,00 | 1,00 | 1,00 |
| Tocopheryl Acetate | 0,05 | 0,20 | 0,10 | - | - | - |
| Benzophenone-2 | 0,01 | 0,01 | 0,01 | 0,05 | 0,05 | 0,05 |
| Colours approved for Cosmetics | 0,0001 | 0,0005 | 0,0010 | - | - | - |
| Parfum | 0,80 | 1,00 | 2,00 | 1,50 | 1,50 | 1,50 |
| Protectate MOD 2 | 0,30 | 0,50 | - | - | - | - |
| Protectate HR | - | - | 0,20 | 0,60 | - | - |
| Jasmol | - | - | - | - | 0,40 | - |
| Phenoxyethanol | - | 1,00 | - | 1,50 | 1,50 | 1,50 |
| Protectate MOD 3 | - | - | - | - | - | 0,30 |
| Aqua | ad 100 | ad 100 | ad 100 | ad 100 | ad 100 | ad 100 |

12. Antitranspirant-PIT-Emulsionen im Pumpdispenser (treibgasfrei)

| | 1 | 2 | 3 | 4 |
|---|---|---|---|---|
| Aluminum chlorohydrate 50% solution | 30,00 | 40,00 | 35,00 | 40,00 |
| Dicaprylyl Ether, z. B. Cetiol OE | 10,00 | 10,00 | 8,00 | 9,00 |
| Glycerin 86% | 5,00 | 3,00 | 5,00 | 3,00 |
| Beheneth-10 | 3,30 | 4,00 | 3,50 | 4,00 |
| Cetearyl Isononanoate | - | - | 4,00 | 5,00 |
| Hexyldecanol / Hexyldecyl Laurate | 3,00 | 5,00 | - | - |
| Parfum | 1,00 | 0,80 | 1,20 | 1,00 |
| Plant Extract | 0,20 | - | 0,50 | - |
| Polysorbate 20 / Linoleic Acid | 0,20 | 0,20 | 0,50 | - |
| Allantoin | 0,10 | - | - | 0,20 |
| Protectate MOD 2 | 0,30 | 0,50 | - | - |
| Protectate HR | - | - | 0,20 | - |
| Aqua | ad 100 | ad 100 | ad 100 | ad 100 |

13. Klare Antitranspirant-Gele

| | 1 | 2 | 3 | 4 | 5 |
|---|---|---|---|---|---|
| Propylene Glycol 1,2 | 18,00 | 23,00 | 18,00 | 20,00 | 18,00 |
| Aluminum Chlorohydrate 50% solution | 40,00 | 40,00 | 40,00 | 40,00 | 40,00 |
| Cyclopentasiloxane | 14,20 | 14,20 | 14,20 | 14,20 | 14,20 |
| Alcohol Denat. | 5,00 | 10,00 | 8,00 | 10,00 | 5,00 |
| Bis-PEG/PPG-14/14 Dimethicone, Cyclomethicone, z. B. DC 5225 | 3,50 | 2,50 | 3,20 | 3,00 | 3,50 |
| Parfum | 0,60 | 0,60 | 1,00 | 1,30 | 0,60 |
| Plant Extract | 0,50 | - | - | - | 0,50 |
| Allantoin | - | 0,10 | - | - | - |
| 2-Ethylhexylglycerin | 0,30 | - | - | 0,50 | 0,30 |
| Phenoxyethanol | 0,50 | - | - | 1,00 | 0,50 |
| Protectate MOD 2 | 0,30 | 0,50 | - | - | - |
| Protectate HR | - | - | 0,20 | - | - |
| Jasmol | - | - | - | 0,40 | 0,23 |
| Aqua | ad 100 | ad 100 | ad 100 | ad 100 | ad 100 |

Um klare Gele zu erzielen, ist der Brechungsindex der Wasserphase dem Brechungsindex der Ölphase anzupassen. Wasser bzw. Propylenglycol dienen als Variable.
14. Klare Deodorant-Gele

| | 1 | 2 | 3 | 4 | 5 |
|---|---|---|---|---|---|
| Alcohol Denat. | 30,00 | 40,00 | 50,00 | 60,00 | 60,00 |
| Ceteareth-12 | 1,50 | - | 2,00 | - | - |
| Ceteareth-30 | 2,50 | - | 2,00 | - | - |
| PEG-40 Hydrogenated Castor Oil | - | 3,00 | - | 2,00 | 2,00 |
| Carbomer | 0,30 | 0,50 | 0,80 | 1,00 | 1,00 |
| Neutralisation Agent | q.s.* | q.s.* | q.s.* | q.s.* | q.s.* |
| Parfum | 0,60 | 0,60 | 1,00 | 1,30 | 1,30 |
| Plant Extract | 0,50 | - | 0,20 | - | - |
| Ethylhexylglycerin | - | 0,30 | - | 1,20 | 1,20 |
| Protectate MOD 2 | 0,30 | 0,50 | - | - | - |
| Protectate HR | - | - | 0,40 | 0,60 | - |
| Protectate MOD 3 | - | - | - | - | 0,20 |
| Phenoxyethanol | - | 1,00 | - | 1,80 | 1,00 |
| Aqua | ad 100 | ad 100 | ad 100 | ad 100 | ad 100 |

Das Verdickungsmittel (Carbomer, z. B. Carbopol 980) ist mit einem geeigneten Neutralisierungsmittel (Triethanolamin, 2-Amino-2-methylpropanol-1 (AMP), Natriumhydroxid, Lithiumhydroxid) auf den gewünschten pH-Wert einzustellen.
15. Antitranspirant-Creme

| | 1 | 2 | 3 | 4 |
|---|---|---|---|---|
| Aluminum Chlorohydrate 50% solution | 40,00 | 40,00 | 35,00 | 45,00 |
| Glyceryl Stearate, z. B. Cutina MD | 5,00 | 4,50 | 5,50 | 6,00 |
| Cetyl Alcohol | 2,00 | - | 3,00 | 1,50 |
| Behenyl Alcohol | 1,50 | 4,00 | 3,50 | 5,00 |
| Dimethicone | 2,00 | 1,50 | 2,50 | 3,00 |
| Ceteareth-12 | 1,50 | 2,00 | 2,50 | 1,30 |
| Ceteareth-20 | 1,50 | 2,00 | 2,50 | 1,30 |
| Hexyldecanol /Hexyldecyl Laurate | 3,00 | 4,00 | 2,50 | 2,40 |
| Cyclopentasiloxane | 1,50 | 3,00 | 2,00 | 1,00 |
| Plant Extract | 0,20 | 0,50 | - | - |
| Tocopheryl Acetate | 0,05 | 0,25 | - | - |
| Parfum | 0,80 | 1,00 | 1,50 | 2,00 |
| Allantoin | 0,10 | 0,10 | - | - |
| Preservative System | 0,05 | 0,50 | 0,50 | 0,50 |
| 2-Ethylhexylglycerin | 0,30 | - | - | 1,20 |
| Phenoxyethanol | - | - | - | 1,50 |
| Protectate MOD 2 | 0,30 | 0,50 | - | - |
| Protectate HR | - | - | 0,20 | - |
| Jasmol | - | - | - | 0,50 |
| Aqua | ad 100 | ad 100 | ad 100 | ad 100 |

16. Tränklösungen für Deodorant-Tücher

| | 1 | 2 | 3 | 4 | 5 | 6 |
|---|---|---|---|---|---|---|
| Alcohol Denat. | 50,00 | 55,00 | 60,00 | 40,00 | 50,00 | 55,00 |
| Triethylcitrate | 2,50 | 3,50 | 4,00 | 3,00 | 2,50 | 3,50 |
| PEG-40 Hydrogenated Castor Oil | 1,00 | 0,50 | 0,50 | 2,00 | 1,00 | 0,50 |
| 2-Ethylhexylglycerin | 0,10 | 0,30 | - | - | 0,20 | 0,50 |
| Tocopheryl Acetate | 0,05 | 0,20 | 0,10 | - | 0,05 | 0,20 |
| Benzophenone-2 | 0,01 | 0,01 | 0,01 | 0,05 | 0,01 | 0,01 |
| Colours approved for Cosmetics | 0,0001 | 0,0005 | 0,0010 | - | 0,0001 | 0,0005 |
| Parfum | 0,80 | 1,00 | 2,00 | 1,50 | 0,80 | 1,00 |
| Protectate MOD 2 | 0,30 | 0,50 | - | - | - | - |
| Protectate HR | - | - | 0,20 | 0,60 | - | - |
| Protectate MOD 3 | - | - | - | - | 0,30 | 0,50 |
| Phenoxyethanol | - | 1,00 | - | - | - | 1,00 |
| Aqua | ad 100 | ad 100 | ad 100 | ad 100 | ad 100 | ad 100 |

17. Tränklösungen (PIT-Emulsionen) für Antitranspirant-Tücher

| | 1 | 2 | 3 | 4 |
|---|---|---|---|---|
| Aluminum chlorohydrate 50% solution | 30,00 | 40,00 | 35,00 | 40,00 |
| Dicaprylyl Ether | 10,00 | 10,00 | 8,00 | 9,00 |
| Glycerin 86% | 5,00 | 3,00 | 5,00 | 3,00 |
| Beheneth-10 | 3,30 | 4,00 | 3,50 | 4,00 |
| Cetearyl Isononanoate | - | - | 4,00 | 5,00 |
| Hexyldecanol / Hexyldecyl Laurate | 3,00 | 5,00 | - | - |
| Parfum | 1,00 | 0,80 | 1,20 | 1,00 |
| Plant Extract | 0,20 | - | 0,50 | - |
| Polysorbate 20 /Linoleic Acid | 0,20 | 0,20 | 0,50 | - |
| Allantoin | 0,10 | - | - | 0,20 |
| 2-Ethylhexylglycerin | 0,30 | - | - | - |
| Protectate MOD 2 | 0,30 | 0,50 | - | - |
| Protectate HR | - | - | 0,20 | - |
| Preservative System | 0,50 | 0,20 | 1,00 | 0,50 |
| Aqua | ad 100 | ad 100 | ad 100 | ad 100 |

### Liste der verwendeten Rohstoffe

| Carbopol 980 | Carbomer | Noveon |
|---|---|---|
| Cetiol^{®} OE | DICAPRYLYL ETHER | Cognis |
| Cetiol^{®} PGL | Hexyldecanol, Hexyldecyl Laurate | Cognis |
| Citrus Liquid Extract | CITRUS AURANTIUM DULCIS (ORANGE) SEED EXTRACT, GLYCERIN (Citrusfruchtsamenextrakt in Glycerin im Verhältnis 1:1) | Centroflora, Brazil |
| Cremophor^{®} RH 455 | hydriertes Rizinusöl mit 40 EO, Propylenglykol-haltig | BASF |
| Crodafos^{®}SG | PPG-5-Ceteth-10 Phosphate | Croda Oleochemicals |
| Cutina GMS | Glyceryl Stearate (Mschung aus Glycerylmono- und distearat) | Cognis |
| Cutina MD | Glyceryl Stearate | Cognis |
| Cutina^{®} E 24 PF | PEG-20 GLYCERYL STEARATE | Cognis |
| Cutina^{®} HR | gehärtetes Rizinusöl | Cognis |
| DC^{®} 245 | Cyclopentasiloxan | Dow Corning |
| DC^{®} 345 | Cyclopentasiloxan, Cyclohexasiloxan | Dow Corning |
| Lanette^{®} 22 | Behenyl Alcohol | Cognis |
| Lanette^{®} O | Cetearyl Alcohol | Cognis |
| Lorol^{®} C 18 | Stearylalkohol | Cognis |
| Novata AB | Cocoglycerides | Cognis |
| Pemulen TR 1 | Acrylates/C10-30 Alkyl Acrylate Cross-polymer | Noveon |
| Sensiva^{®} SC 50 | 2-Ethylhexylglycerinether | Schülke & Mayr |
| Sepigel^{®}305 | Polyacrylamide, C₁₃-C₁₄ Isoparaffin, Laureth-7 | SEPPIC |
| Stenol 16/18 | Cetearylalkohol | Cognis |
| Ucon Fluid^{®} AP | PPG-14 BUTYL ETHER | Amerchol (Union Carbide) |

## Patentansprüche

1. Verwendung von mindestens einer Substanz, die den an der Körpergeruchsbildung beteiligten Keim Staphylococcus hominis inhibiert, in einer kosmetischen Deodorant- oder Antitranspirant-Zusammensetzung zur Verringerung des Körpergeruchs.

2. Verwendung gemäß Anspruch 1, **dadurch gekennzeichnet, dass** die den an der Körpergeruchsbildung beteiligten Keim Staphylococcus hominis inhibierende(n) Substanz(en) ausgewählt ist/sind aus Derivaten von C₃-C₉-Polyolen, Phenolderivaten, monocyclischen Sesquiterpenen vom Bisabolen- oder Bisabolan-Typ, primären Alkanolen mit einer Alkylkettenlänge von C₃ - C₈ und einem Benzyl- oder Phenylsubstituenten, Aluminiumchlorohydrat Aluminiumzirconiumchlorohydrat, Extrakten aus Citrusfruchtsamen, den Riechstoffgemischen Protectate HR, Protectate MOD 2 und Protectate MOD 3, sowie Mischungen dieser Substanzen.

3. Verwendung gemäß Anspruch 2, **dadurch gekennzeichnet, dass** die Derivate von C₃-C₉-Polyolen ausgewählt sind aus Monoalkyl-C₃-C₉-Polyolethern.

4. Verwendung gemäß Anspruch 3, **dadurch gekennzeichnet, dass** die Monoalkyl-C₃-C₉-Polyolether ausgewählt sind aus α-Monohexylglycerinether, α-Mono-(2-ethylhexyl)glycerinether, α-Mono-(2-ethyloctyl)glycerinether und α-Mono-(2-ethyldecyl)glycerinether.

5. Verwendung gemäß Anspruch 2, **dadurch gekennzeichnet, dass** die Phenolderivate ausgewählt sind aus Phenoxyethanol, 4-Methoxybenzylalkohol (para-Anisalkohol) und 5-Methyl-2-isopropylphenol (Thymol) sowie Mischungen dieser Substanzen.

6. Verwendung gemäß Anspruch 2, **dadurch gekennzeichnet, dass** die monocyclischen Sesquiterpene vom Bisabolen- oder Bisabolan-Typ ausgewählt sind aus (-)-α-Bisabolol, (R)-(E)-α-Bisabolen, (S)-(E)-α-Bisabolen, (R)-(Z)-α-Bisabolen, (S)-(Z)-α-Bisabolen, (R)-β-Bisabolen, (S)-β-Bisabolen, (E)-y-Bisabolen, (Z)-y-Bisabolen, Zingiberen, (R)-(-)-α-Curcumen, (S)-(+)-α-Curcumen, (S)-(+)-β-Curcumen und (S)-(+)-y-Curcumen.

7. Verwendung gemäß Anspruch 2, **dadurch gekennzeichnet, dass** die primären Alkanole mit einer Alkylkettenlänge von C₃ - C₈ und einem Benzyl- oder Phenylsubstituenten ausgewählt sind aus 2-Methyl-5-phenylpentan-1-ol und 2-Benzylheptan-1-ol.

8. Verwendung gemäß einem der vorstehenden Patentansprüche, **dadurch gekennzeichnet, dass** die den an der Körpergeruchsbildung beteiligten Keim Staphylococcus hominis inhibierenden Substanzen ausgewählt sind aus Mischungen, die a) 0,1 - 2,0 Gew.-%, besonders bevorzugt 0,25 - 1,5 Gew.-% und außerordentlich bevorzugt 0,25 - 1,0 Gew.-% α-Mono-(2-ethylhexyl)glycerinether, weiterhin b) 0,1 - 2,5 Gew.- %, insbesondere 0,5 - 2,0 Gew.-% und außerordentlich bevorzugt 1,0 - 1,5 Gew.-% Phenoxyethanol und weiterhin c) 0,03 - 1,0 Gew.-%, bevorzugt 0,15 - 0,5 Gew.-% und besonders bevorzugt 0,3 - 0,4 Gew.-% Jasmol, jeweils bezogen auf das Gewicht der gesamten kosmetischen Zusammensetzung, enthalten.

9. Verwendung gemäß einem der vorstehenden Patentansprüche, **dadurch gekennzeichnet, dass** die den an der Körpergeruchsbildung beteiligten Keim Staphylococcus hominis inhibierenden Substanzen ausgewählt sind aus Mischungen aus 0,1-2,0 Gew.-% α-Mono-(2-ethylhexyl)glycerinether, 0,1 - 2,5 Gew.-% Phenoxyethanol und 0,001 - 3,0 Gew.-% (-)-α-Bisabolol, wobei alle Mengenangaben auf das Gewicht der gesamten kosmetischen Zusammensetzung bezogen sind.

10. Verwendung gemäß einem der vorstehenden Patentansprüche, **dadurch gekennzeichnet, dass** zusätzlich Triethylcitrat enthalten ist.

11. Verwendung gemäß einem der vorstehenden Patentansprüche, **dadurch gekennzeichnet, dass** die den an der Körpergeruchsbildung beteiligten Keim Staphylococcus hominis inhibierenden Substanzen ausgewählt sind aus Mischungen, die a) 0,1 - 1,2 Gew.-%, bevorzugt 0,5 - 1,0 Gew.-% α-Mono-(2-ethylhexyl)glycerinether, weiterhin b) 0,001 - 3,0 Gew.-% (-)-α-Bisabolol, weiterhin c) 0,1 - 2,0 Gew.-% Phenoxyethanol und weiterhin d) 0,1 - 6 Gew.-%, insbesondere 0,5 - 4 Gew.-% Triethylcitrat, jeweils bezogen auf das Gewicht der gesamten kosmetischen Zusammensetzung, enthalten.

12. Verwendung gemäß einem der vorstehenden Patentansprüche, **dadurch gekennzeichnet, dass** die den an der Körpergeruchsbildung beteiligten Keim Staphylococcus hominis inhibierenden Substanzen ausgewählt sind aus Mischungen, die a) 0,1 - 1,5 Gew.-%, bevorzugt 0,25 - 1,0 Gew.-% α-Mono-(2-ethylhexyl)glycerinether, weiterhin b) 0,5 - 2,0 Gew.-% Phenoxyethanol, weiterhin c) 0,03 - 0,75 Gew.-%, bevorzugt 0,075 - 0,5 Gew.-% Jasmol und weiterhin d) 0,1 - 6 Gew.-%, insbesondere 0,5 - 4 Gew.-% Triethylcitrat, jeweils bezogen auf das Gewicht der gesamten kosmetischen Zusammensetzung, enthalten.

13. Verfahren zur Verringerung von Körpergeruch mittels Inhibierung des an der Körpergeruchsbildung beteiligten Keims Staphylococcus hominis auf der Haut, **dadurch gekennzeichnet, dass** eine kosmetische Deodorant- oder Antitranspirant-Zusammensetzung, enthaltend mindestens eine den an der Körpergeruchsbildung beteiligten Keim Staphylococcus hominis inhibierende Substanz auf die Haut, insbesondere auf die Haut der Achselhöhlen, aufgetragen wird.

14. Verfahren zur Verringerung von Körpergeruch nach Anspruch 13, **dadurch gekennzeichnet, dass** die den an der Körpergeruchsbildung beteiligten Keim Staphylococcus hominis inhibierende(n) Substanz(en) ausgewählt ist/sind aus Derivaten von C₃₋C₉-Polyolen, Phenolderivaten, monocyclischen Sesquiterpenen vom Bisabolen- oder Bisabolan-Typ, primären Alkanolen mit einer Alkylkettenlänge von C₃ - C₈ und einem Benzyl- oder Phenylsubstituenten, Aluminiumchlorohydrat, Aluminiumzirkoniumchlorohydrat, Extrakten aus Citrusfruchtsamen, den Riechstoffgemischen Protectate HR, Protectate MOD 2 und Protectate MOD 3 der Firma Symrise sowie Mischungen dieser Substanzen.

15. Verfahren zur Verringerung von Körpergeruch gemäß einem der Ansprüche 13 oder 14, **dadurch gekennzeichnet, dass** die den an der Körpergeruchsbildung beteiligten Keim Staphylococcus hominis inhibierende(n) Substanz(en) hinsichtlich ihrer Menge und/oder ihrer Art geschlechtsspezifisch eingesetzt werden.

16. Verfahren zur Verringerung von Körpergeruch gemäß Anspruch 15, **dadurch gekennzeichnet, dass** die den an der Körpergeruchsbildung beteiligten Keim Staphylococcus hominis inhibierende(n) Substanz(en) zur Verringerung von Körpergeruch beim Mann eingesetzt wird/werden.
